(19) Europäisches Patentamt
European Patent Office
Office européen des brevets

(11) Veröffentlichungsnummer: **0 386 733 B1**

# (12) EUROPÄISCHE PATENTSCHRIFT

(45) Veröffentlichungstag der Patentschrift: **05.07.95**

(21) Anmeldenummer: **90104352.1**

(22) Anmeldetag: **07.03.90**

(51) Int. Cl.[6]: **C12N 15/12**, C12P 21/02, C12N 1/21, G01N 33/577, //(C12N1/21,C12R1:04)

## (54) Für das Plazentaprotein 9 (PP9) kodierende cDNA, ihre Isolierung und Verwendung.

(30) Priorität: **10.03.89 DE 3907744**

(43) Veröffentlichungstag der Anmeldung:
**12.09.90 Patentblatt 90/37**

(45) Bekanntmachung des Hinweises auf die Patenterteilung:
**05.07.95 Patentblatt 95/27**

(84) Benannte Vertragsstaaten:
**AT BE CH DE DK ES FR GB IT LI LU NL SE**

(56) Entgegenhaltungen:
**EP-A- 0 037 963**

**ARCHIV für Gynäkologie, Band 223, 1977 (München) H. BOHN et al. "Isolierung und Charakterisierung des Plazenta-Proteins PP5" Seite 179-186**

(73) Patentinhaber: **BEHRINGWERKE Aktiengesellschaft**
**Postfach 1140**
**D-35001 Marburg (DE)**

(72) Erfinder: **Grundmann, Ulrich, Dr.**
**Am Pfahltor 7**
**D-3551 Lahntal-Grossfelden (DE)**
Erfinder: **AMANN Egon, Hoechst Japan Ltd**
**1-3-2 Minamidai**
**Kawagoe, Saitama 350 (JP)**

EP 0 386 733 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Isolierung der cDNA, die für das plazentaspezifische Protein 9 (PP9) kodiert und deren Verwendung zur gentechnischen Herstellung von PP9. PP9 hat nach der Beschreibung in EP-B1-0 037 963 folgende Eigenschaften:

a) ein Gehalt an Kohlenhydraten von 5,57 ± 1,35% und davon: Hexosen 4,9 ± 1,0%; Hexosamine 0,1 ± 0,1%; Fucose 0,07 ± 0,05%; Neuraminsäure 0,5 ± 0,2%;

b) einen Sedimentationskoeffizienten

$$s^{o}_{20.w} \text{ von}$$

3,2 ± 0,2 S;

c) ein in der Ultrazentrifuge bestimmtes Molekulargewicht von 35 100 ± 3 800;

d) ein im Natriumdodecylsulfat (SDS)-haltigen Polyacrylamidgel bestimmtes Molekulargewicht von 40 000 ± 4 000;

e) einen Extinktionskoeffizienten

$$E^{1\%}_{1\ cm}$$

(280 nm) von 14,6 ± 1,0;

f) eine elektrophoretischen Beweglichkeit im Bereich der $\beta_1$-Globuline;

g) einen isoelektrischen Punkt im Bereich von pH 5,0-6,8.

Da die in o.g. Patentschrift beschriebene konventionelle Isolierung von PP9 sehr aufwendig ist, stellte sich deshalb die Aufgabe, das für PP9 kodierende Gen zu isolieren, um die gentechnische Herstellung von PP9 zu ermöglichen.

Überraschenderweise wurden fünf individuelle Klone gewonnen (PP9-10, PP9-353, PP9-357, PP9-361 und PP9-362), als in einer Expressionsgenbank mit Antikörpern gegen ein anderes, nicht als ähnlich zu PP9 bekanntes Plazentaprotein PP5 (Bohn und Winkler, Arch. Gynäk. 223, 179-186, 1977) gesucht wurde. Diese Klone enthalten die gesamte cDNA oder Teile davon. Bei der Sequenzierung von PP9-10 wurde gefunden, daß die cDNA von einer unvollständig prozessierten, heterogenen Kern-RNA (HnRNA) zu stammen scheint, da hier inmitten der - allerdings nicht ganz vollständigen - für PP9 kodierenden Sequenz ein etwa 600 bp großes Intron vorliegt und am 3′ Ende keine Poly(A)-Sequenz vorhanden ist. Die anderen vier Klone tragen alle kein Intron, wohl aber die Poly(A)-Sequenz. Die cDNA für PP5 konnte mit obigem Verfahren nicht isoliert werden.

Die Sequenzierungsdaten liefern das Ergebnis, daß die prozessierte gesamte cDNA von PP9 1394 Basenpaare (bp) lang ist und für ein Protein mit 316 Aminosäuren (AS) kodiert (Tab. 1). Das Molekulargewicht von 35853d und die AS-Zusammensetzung stimmen sehr gut mit dem in vorgenannter Patentschrift enthaltenen Angaben überein. (siehe Tab. 2).

Tab. 1

```
          10                             30                             50
GAGCGCAGCAGCCATGGCAAGCCGTCTCCTGCTCAACAACGGCGCCAAGATGCCCATCCT
                M  A  S  R  L  L  L  N  N  G  A  K  M  P  I  L

          70                             90                            110
GGGGTTGGGTACCTGGAAGTCCCCTCCAGGGCAGGTGACTGAGGCCGTGAAGGTGGCCAT
 G  L  G  T  W  K  S  P  P  G  Q  V  T  E  A  V  K  V  A  I

         130                            150                            170
TGACGTCGGGTACCGCCACATCGACTGTGCCCATGTGTACCAGAATGAGAATGAGGTGGG
 D  V  G  Y  R  H  I  D  C  A  H  V  Y  Q  N  E  N  E  V  G

         190                            210                            230
GGTGGCCATTCAGGAGAAGCTCAGGGAGCAGGTGGTGAAGCGTGAGGAGCTCTTCATCGT
 V  A  I  Q  E  K  L  R  E  Q  V  V  K  R  E  E  L  F  I  V

         250                            270                            290
CAGCAAGCTGTGGTGCACGTACCATGAGAAGGGCCTGGTGAAAGGAGCCTGCCAGAAGAC
 S  K  L  W  C  T  Y  H  E  K  G  L  V  K  G  A  C  Q  K  T

         310                            330                            350
ACTCAGCGACCTGAAGCTGGACTACCTGGACCTCTACCTTATTCACTGGCCGACTGGCTT
 L  S  D  L  K  L  D  Y  L  D  L  Y  L  I  H  W  P  T  G  F

         370                            390                            410
TAAGCCTGGGAAGGAATTTTTCCCATTGGATGAGTCGGGCAATGTGGTTCCCAGTGACAC
 K  P  G  K  E  F  F  P  L  D  E  S  G  N  V  V  P  S  D  T

         430                            450                            470
CAACATTCTGGACACGTGGGCGGCCATGGAAGAGCTGGTGGATGAAGGGCTGGTGAAAGC
 N  I  L  D  T  W  A  A  M  E  E  L  V  D  E  G  L  V  K  A

         490                            510                            530
TATTGGCATCTCCAACTTCAACCATCTCCAGGTGGAGATGATCTTAAACAAACCTGGCTT
 I  G  I  S  N  F  N  H  L  Q  V  E  M  I  L  N  K  P  G  L

         550                            570                            590
GAAGTATAAGCCTGCAGTTAACCAGATTGAGTGCCACCCATATCTCACTCAGGAGAAGTT
 K  Y  K  P  A  V  N  Q  I  E  C  H  P  Y  L  T  Q  E  K  L

         610                            630                            650
AATCCAGTACTGCCAGTCCAAAGGCATCGTGGTGACCGCCTACAGCCCCCTCGGCTCTCC
 I  Q  Y  C  Q  S  K  G  I  V  V  T  A  Y  S  P  L  G  S  P

         670                            690                            710
TGACAGGCCCTGGGCCAAGCCCGAGGACCCTTCTCTCCTGGAGGATCCCAGGATCAAGGC
 D  R  P  W  A  K  P  E  D  P  S  L  L  E  D  P  R  I  K  A

         730                            750                            770
GATCGCAGCCAAGCACAATAAAACTACAGCCCAGGTCCTGATCCGGTTCCCCATGCAGAG
 I  A  A  K  H  N  K  T  T  A  Q  V  L  I  R  F  P  M  Q  R

         790                            810                            830
GAACTTGGTGGTGATCCCCAAGTCTGTGACACCAGAACGCATTGCTGAGAACTTTAAGGT
```

```
N  L  V  V  I  P  K  S  V  T  P  E  R  I  A  E  N  F  K  V

        850                     870                     890
CTTTGACTTTGAACTGAGCAGCCAGGATATGACCACCTTACTCAGCTACAACAGGAACTG
 F  D  F  E  L  S  S  Q  D  M  T  T  L  L  S  Y  N  R  N  W

        910                     930                     950
GAGGGTCTGTGCCTTGTTGAGCTGTACCTCCCACAAGGATTACCCCTTCCATGAAGAGTT
 R  V  C  A  L  L  S  C  T  S  H  K  D  Y  P  F  H  E  E  F

        970                     990                    1010
TTGAAGCTGTGGTTGCCTGCTCGTCCCCAAGTGACCTATACCTGTGTTTCTTGCCTCATT

       1030                    1050                    1070
TTTTTCCTTGCAAATGTAGTATGGCCTGTGTCACTCAGCAGTGGGACAGCAACCTGTAGA

       1090                    1110                    1130
GTGGCCAGCGAGGGCGTGTCTAGCTTGATGTTGGATCTCAAGAGCCCTGTCAGTAGAGTA

       1150                    1170                    1190
GAAGTCTCTTCCAGTTTGCTTTGCCCTTCTTTCTACCCTGCTGGGGAAAGTACAACCTGA

       1210                    1230                    1250
ATACCCTTTTCTGACCAAAGAGAAGCAAAATCTACCAGGTCAAAATAGTGCCACTAACGG

       1270                    1290                    1310
TTGAGTTTTGACTGCTTGGAACTGGAATCCTTTCAGCAAGACTTCTCTTTGCCTCAAATA

       1330                    1350                    1370
AAAAGTGCTTTTGTGAAAAAAAAAAAAAAAAAAAAAAAAAAAAAAAAAAAAAAAAAAAAA

       1390
AAAAAAAAAAAAAA
```

Tab.2

| Aminosäurezusammensetzung von PP9 | | | |
|---|---|---|---|
| Aminosäure | Anzahl | cDNA | PP9 (EP-B1-0 037 963) |
| A = Ala | 19 | 6.013 | 5.69 |
| B = Asx | 0 | 0.000 | 0.00 |
| C = Cys | 7 | 2.215 | 2.29 |
| D = Asp | 15 | 4.747 | 10.30 * |
| E = Glu | 23 | 7.278 | 11.18 ** |
| F = Phe | 11 | 3.481 | 3.87 |
| G = Gly | 16 | 5.063 | 5.50 |
| H = His | 9 | 2.848 | 2.58 |
| I = Ile | 18 | 5.696 | 5.28 |
| K = Lys | 25 | 7.911 | 8.04 |
| L = Leu | 34 | 10.759 | 10.55 |
| M = Met | 6 | 1.899 | 1.47 |
| N = Asn | 15 | 4.747 | 10.30 * |
| P = Pro | 20 | 6.329 | 6.17 |
| Q = Gln | 13 | 4.114 | 11.18 ** |
| R = Arg | 11 | 3.481 | 3.49 |
| S = Ser | 17 | 5.380 | 5.70 |
| T = Thr | 15 | 4.747 | 4.29 |
| V = Val | 25 | 7.911 | 7.12 |
| W = Trp | 6 | 1.899 | 2.36 |
| Y = Tyr | 11 | 3.481 | 3.95 |
| Z = Glx | 0 | 0.000 | 0.00 |
| A+G | 35 | 11.076 | 11.19 |
| S+T | 32 | 10.127 | 9.99 |
| D+E | 38 | 12.025 | -- |
| D+E+N+Q | 66 | 20.886 | -- |
| H+K+R | 45 | 14.241 | 14.11 |
| D+E+H+K+R | 83 | 26.266 | -- |
| I+L+M+V | 83 | 26.266 | 24.42 |
| F+W+Y | 28 | 8.861 | 10.18 |

* = Summe aus Asp + Asn

** = Summe aus Glu + Gln

Die Figur zeigt schematisch die Lage von lambda gt11-10 zu lambda gt11-362. Zwei Klone zeigen einen Basenaustausch:
PP9-361 an Position 255 (C statt G; AS Q statt E) und
PP9-362 an Position 925 (G statt A; AS R statt K).

Durch die hiermit vorliegende cDNA-Sequenz von PP9 wurden Vergleiche mit anderen bekannten Nukleinsäuresequenzen möglich. Es wurde gefunden, daß Homologien zur DNA-Sequenz der Aldose-Reduktase von der Ratte (Carper et al. (1987) FEBS Letters 220, 209-213) sowie zu den Genen von rho-Kristallin und der Aldehyd-Reduktase bestehen. Möglicherweise gehoren diese Proteine zur gleichen "Superfamily" wie die Aldehyd-Reduktase; mit großer Wahrscheinlichkeit ist PP9 identisch mit der humanen Aldose Reduktase.

Erfindungsgemäß kann die kodierende cDNA mittels geeigneter Expressionssysteme dazu genutzt werden, PP9 zu exprimieren. Durch die Auswahl des Wirts kann weiterhin die Form der Modifikation von PP9 beeinflußt werden. So findet in Bakterien keine, in Hefezellen eine andere Glykosylierung als in höheren eukaryotischen Zellen statt. Besonders vorteilhaft erfolgt die Expression von PP9 in E.coli mit dem Expressionsvektor pTrc99c bzw. pTacT7L (siehe Beispiele).

In Kenntnis der Aminosäuresequenz von PP9 ist es möglich, nach konventionellen oder gentechnischen Methoden Aminosäure-Teilsequenzen herzustellen, die als Antigene zur Herstellung von polyklonalen oder monoklonalen Antikörpern dienen. Solche Antikörper sind nicht nur zu diagnostischen Zwecken, sondern auch zur Herstellung von Antikörpersäulen einsetzbar. PP9 kann somit aus Lösungen abgetrennt werden,

die es neben anderen Proteinen enthalten. Mit Hilfe der cDNA bzw. Teilen davon kann man auch auf einfache Weise aus einer genomischen Bank den für PP9 kodierenden genomischen Klon isolieren, mit dessen Hilfe nicht nur eine Expression in eukaryotischen Zellen erleichtert wird, sondern auch weitere diagnostische Aussagen möglich sind.

Aldose-Reduktase (EC1.1.1.21) ist ein NADPH abhängiges Enzym und katalysiert die Reduktion von Aldose zum Zucker Alkohol. Beim Krankheitsbild der Diabetes und der Galaktosämie führt erhöhte Aldose Reduktase Aktivität in einer Reihe von Geweben zu hohen Sorbitol- und Galaktitolspiegel. Dies kann zu z.B. Katarakten in der Linse sowie zu einer Verdickung der Kapillarmembran der Retina führen. Weiterhin sind erhöhte Aldose Reduktase Spiegel als Ursache diabetischer Komplikationen, etwa der Nerven oder der Niere, anzusehen. Aus diesen Gründen wird z. Zt. intensiv an der Entwicklung von Aldose Inhibitoren gearbeitet. Bisher mußte zu diesem Zweck die Aldose Reduktase aufwendig aus tierischen Geweben und Organen isoliert werden, was im übrigen in sehr geringen Ausbeuten resultierte. Die Möglichkeit der Expression der Aldose Reduktase in Escherichia coli (E.coli) ermöglicht nun den Aufbau eines Testsystems mittels bakterieller Extrakte und ersetzt somit die Abhängigkeit von tierischen Organen und Geweben. Das Testverfahren wird insgesamt erheblich vereinfacht.

Die Erfindung ist ferner in den Patentansprüchen definiert und in folgenden Beispielen weiter ausgeführt.

Soweit nicht im Text erläutert, werden die folgenden Abkürzungen verwendet:

**EDTA** = Natrium-ethylendiamin-tetraacetat
**SDS** = Natrium-dodecylsulfat
**DTT** = Dithiothreitol
**BSA** = Rinderserumalbumin
**IPTG** = Isopropylthiogalaktosid

## Beispiele

### 1. Screening einer Expressions-cDNA Bank aus humaner Plazenta mit anti-PP5 Antikörpern

Eine Expressions-cDNA Bank im Phagen lambda gt11 der Fa. Genofit GmbH, Heidelberg wurde mit einer Dichte von etwa 30 000 PFU pro Agarplatte (13,5 cm Durchmesser) ausplattiert. Dazu wurden kompetente Zellen des E.coli Stammes y1090 (ATCC 37197) (R.A. Young and R.W. Davis, Science Vol. 222, 778-782/1983) mit den Phagen 30 min. bei 37°C infiziert und dann auf L-Broth-Platten in Top-Agar ausplattiert. Die Platten wurden 4 h bei 42°C inkubiert, danach mit je einem trockenen Nitrocellulosefilter (Schleicher und Schuell, BA 85, Ref.Nr. 401124) bedeckt. Die Filter waren zuvor mit 10 mM IPTG in Wasser gesättigt worden. Die Platten mit den Filtern wurden erneut 4 h bei 37°C inkubiert. Bevor die Filter wieder abgenommen wurden, wurden Filter und Platte zugleich mit einer rußgeschwärzten Nadel markiert. Die Filter wurden dann in TBST (10 mM Tris-HCl, pH 8,0 150 mM NaCl, 0,05% Tween 20 und 5% Magermilchpulver) über Nacht bei 4°C inkubiert. Die Filter wurden anschließend noch dreimal für 10 min in TBST bei Raumtemperatur gewaschen und dann mit anti-PP5 Kaninchen Antikörpern in 15 ml TBST pro Filter bei Raumtemperatur 1 h inkubiert. (Die Lösung der Antikörper war zuvor 1:200 verdünnt worden und mit nichtrekombinanten lambda gt11 lysierten E.coli Zellen auf Nitrocellulose Filtern 1 h abgesättigt worden). Nach der Inkubation mit dem primären Antikörper wurden die Filter 4 x 10 min. mit TBST gewaschen. Nunmehr wurden die Filter mit dem sekundären Anti-Kaninchen Antikörper, der mit alkalischer Phosphatase konjugiert war (Fa. Promega, USA - Vertrieb durch Fa. Atlanta, Heidelberg) und zuvor 1:5000 in TBST verdünnt wurde, unter Schütteln 1 h inkubiert. Die Filter wurden danach erneut 4 x 10 min. mit TBST gewaschen.

Schließlich erfolgte die Farbreaktion, um die PP9-positiven Klone, an die primärer wie sekundärer Antikörper gebunden waren, unter Reaktion der alkalischen Phosphatase und eines Farbreagenzes (Proto-Blot System der Fa. Protogen) sichtbar zu machen. Pro Farbreaktion wurden für einen Nitrocellulosefilter 15 ml AP Puffer (100 mM Tris-HCl, pH 9,5 100 mM NaCl, 5 mM $MgCl_2$) mit 99 µl NBT-(Nitro Blau Tetrazolium) Substrat (50 mg/ml in 70% Dimethylformamid) und 49,5 µl BCIP-(5-Brom-4-Chlor-3-Indolyl-Phosphat) Substrat (50 mg/ml in 70% Dimethylformamid) versetzt. Die Filter wurden im Dunkeln in der Farblösung etwa 20 min. bis 1 h so lange geschwenkt, bis positive Plaques eine ausreichende Blaufärbung zeigten. Die Farbreaktion wurde beendet durch Eintauchen der Filter in eine Stop-Lösung (20 mM Tris-HCl, pH 8,0 und 5 mM EDTA).

Positive Signale wurden den Plaques auf der entsprechenden Agarplatte zugeordnet. Die Plaques wurden mit einer Pasteurpipette ausgestanzt, in 1 ml SM Puffer (10 mM Tris-HCl pH 7,5, 10 mM $MgCl_2$) resuspendiert und vereinzelt, bis ein einzelner, positiver Plaque vorhanden war. Die positiv reagierenden

Klone PP9-10, PP9-353, PP9-357, PP9-361 und PP9-362 wurden erhalten.

**2. DNA-Sequenzanalyse**

Die vorstehend genannten Phagenklone wurden vermehrt und ihre DNA jeweils extrahiert. Das jeweilige EcoRI-Fragment wurde isoliert und in die EcoRI-Stelle des Bluescript M13 Vektors (Stratagene, San Diego, CA, USA) einligiert. Die Sequenzanalyse erfolgte mittels der enzymatischen Dideoxymethode nach Sanger et al. (Proc.Natl.Acad.Sci.USA 74, (1977);5463-5467). Die Sequenz zeigt einen offenen Leserahmen und kodiert für ein Protein mit maximal 316 Aminosäuren.

**3. Expression eines PP9 Fusionsproteins**

pTrc99C (E. Amann et al., Gene 69, (1988), pp 301-315) wurde mit EcoRI verdaut. Der Klon lambda gt11-361 wurde mit EcoRI verdaut und das 1387 bp große EcoRI-Insert mit dem oben beschriebenen pTrc99C Vektorfragment ligiert. Das so erhaltene Plasmid pTrc99C-PP9 ist in der Lage, in E. coli Zellen die Synthese eines ca. 36 kD Proteines zu induzieren. Dieses Protein kann spezifisch mit Hilfe eines monospezifischen Kaninchen Anti-PP9 Antiserums, welches durch Immunisierung mit aus humanen Plazenten isoliertem PP9 erzeugt worden war, immungefällt werden. Ein weiterer Nachweis der PP9-Expression in E.coli Zellen erfolgte mittels Westernblot-Analysen unter Verwendung obigen Serums. In diesem Experiment reagiert nur der Extrakt aus pTrc99C-PP9 plasmidhaltigen, IPTG-induzierten Zellen, wobei wieder eine Proteinbande von ca. 36 kD spezifisch sichtbar wurde.

Plasmidlose E. coli Kontrollextrakte, bzw. nicht mittels IPTG induzierte pTrc99C-PP9-haltige Extrakte reagierten nicht mit dem obengenannten Anti-PP9 Antiserum. Das durch die weiter oben erzeugte Plasmidkonstruktion erzeugte PP9-Fusionsprotein hat folgende N-terminale Aminosäuresequenz, definiert durch nachfolgende Nukleotidsequenz:

```
          Vektor/Linker /
                              5' UT Region /
                                      PP9
          1   2   3   4   5   6   1   2   3
          Met Gly Asn Ser Ala Ala Met Ala Ser
    ...CC ATG GGG AAT TCT GCA GCC ATG GCA AGC
```

Das durch diese Konstruktion definierte PP9 Fusionsprotein trägt zusätzlich zu der durch die PP9 cDNA kodierte vollständige PP9 Aminosäuresequenz N-terminal vorgeschaltet sechs Aminosäuren: vier vektorkodierte Aminosäuren sowie zwei Aminosäuren, die durch die in der PP9 cDNA vorkommende 5′ untranslatierte Region spezifiziert werden. Zur Kontrolle wurde die weiter oben angegebene Konstruktion ebenfalls mit den Expressionsvektoren pTrc99A und pTrc99B (Amann et al. a.a.O.) durchgeführt. Diese Vektoren unterscheiden sich von pTrc99c lediglich in 2 bp (pTrc99A) bwz. 1 bp (pTrc99B), wodurch Verschiebungen des Translations-Leserahmens hervorgerufen werden. Wie zu erwarten, waren weder pTrc99A-PP9 noch pTrc99B-PP9 in der Lage, die Synthese von mit Anti-PP9 Antiseren reagierenden PP9 Proteinen zu induzieren.

**4. Expression des reifen, unfusionierten PP9 Proteins**

Die PP9 cDNA besitzt neben der NcoI-Stelle (5′CCATGG3′) am Initiationscodon eine weitere NcoI-Stelle im Strukturgen. Um eine reife Expression des PP9 Proteins zu erreichen, wurde das 1387 bp große EcoRI Fragment (s.o.) zunächst in den Vektor pMa5-8 (Stanssens et al., 1989) ligiert. Ein Plasmid (pMa5-8-PP9), in dem das EcoRI Fragment in der gewünschten Orientierung vorlag (PP9 ATG Initiationscodon am linken, 5′ distalen Ende) wurde erhalten und vermehrt. Die Plasmid DNA wurde vollständig mit HindIII und partiell

mit NcoI verdaut. Das 1415 bp große NcoI-HindIII Fragment wurde isoliert und in den mit den gleichen Restriktionsenzymen gespaltenen Expressionsvektor pTrc99A zwischen die entsprechenden Stellen ligiert. Das resultierende Plasmid pTrc99A-PP9R ("R" steht für "reif") umfaßt 5535 bp und exprimiert nach IPTG Induktion das reife, unfusionierte PP9 Protein. Die N-terminale Aminosäure-sequenz des PP9 Proteins wird durch folgende Nukleotidsequenz definiert:

```
                 Met Ala Ser Arg Leu
... AGGAAACAGACC ATG GCA AGC CGT CTC ...
```

Das dergestalt exprimierte Protein reagiert mit anti-PP9 Antiseren im Westernblot und in der Immunfällung, wobei ein ca. 36 kD großes Protein nachgewiesen werden kann. Es wurde nun gefunden, daß dieses reife PP9 Protein in das Periplasma der E.coli Zellen transportiert wird und dort seine enzymatische Aktivität (Aldose Reduktase) entfaltet.

Um die Expressionsrate der Aldose Reduktase in E.coli noch zu erhöhen, wurde ein verbesserter Expressionsvektor konstruiert (pTacT7L), der die Ribosomenbindungsstelle des Gens 10 des T7 Phagen verwendet. Es ist bekannt, daß diese Sequenz unmittelbar vor einem heterologen Gen dessen Expressionsrate durch eine effizientere Ribosomenbindung erhöhen kann (Olins et al. (1988), Gene 73, 227-235). pTacT7L beruht im wesentlichen auf dem bekannten Vektor pKK223-3 (Brosius & Holy (1984), Proc. Natl. Acad. Sci. USA 81, 6929-6933), besitzt aber im Gegensatz zu diesem die bereits erwähnte T7 Sequenz unmittelbar vor dem Klonierungslinker. Das oben erwähnte PP9 kodierende 1415 bp lange NcoI-HindIII Fragment wurde in den mit den gleichen Restriktionsenzymen geschnittenen pTacT7L Vektor ligiert. Das resultierende Plasmid pTacT7L-PP9 vermittelt ebenfalls die Expression des unfusionierten PP9 Proteins, jedoch ist die Ausbeute von PP9 ca. 20-fach höher als diejenige, die von pTrc99A-PP9R erzielt wird.

**5. Aldose Reduktase Aktivität des Proteins PP9 nach Expression in E.coli**

Sequenzhomologievergleiche der PP9 cDNA ergab eine 94% Homologie (85% Identität) in der Computeranalyse mit der Aldose Reduktase der Ratte (Carper et al. (1987) FEBS Lett. 220, 209-213). Weitere Homologien wurden entdeckt zum Rho-Crystallin des Froschauges sowie zur Aldehyd Reduktase der Rattenlinse. Dieser Befund läßt vermuten, daß es sich bei PP9 um ein weiteres Mitglied einer grösseren Proteinfamlie und höchstwahrscheinlich um die humane Aldose Reduktase handelt.

Periplasmafraktionen von E.coli K12 W3110lacIQ (pTacT7L-PP9) wurden nach der Methode von Hsiung et al. (1986) (Bio/Technology 4,991-995) hergestellt. Diese Extrakte besitzen eine Aldose Reduktase Aktivität, die in entsprechenden Kontrollextrakten nicht vorhanden ist. Zum Nachweis der Aldose Reduktase Aktivität wurden bekannten Testmethoden (z.B. Kawasaki et al., (1989) Biochim. Biophys. Acta 996,30-36) herangezogen, die auf einer Abnahme der Absorption bei 340 nm, bedingt durch die Oxidation des NADPH im Testansatz, beruht.

**Patentansprüche**

**Patentansprüche für folgende Vertragsstaaten : AT, BE, CH, DE, DK, FR, GB, IT, LI, LU, NL, SE**

1. Nukleotidsequenz gemäß Tabelle 1 oder davon aufgrund der Degeneration des genetischen Codes abgeleitete Sequenz, kodierend für plazenta-spezifisches Protein PP9.

2. Genstruktur, enthaltend eine Nukleinsäure gemäß Anspruch 1.

3. Vektor, enthaltend eine Nukleinsäure nach Anspruch 1.

4. Transformierte Zellen, enthaltend eine Nukleinsäure nach Anspruch 1.

5. Verfahren zur Herstellung von PP9, dadurch gekennzeichnet, daß man eine Nukleinsäure nach Anspruch 1 in ein Expressionssystem einbringt und dort zur Expression bringt.

6. Diagnostikum, das eine Nukleinsäure nach Anspruch 1 enthält.

**Patentansprüche für folgenden Vertragsstaat : ES**

1. Verfahren zur Herstellung von PP9, dadurch gekennzeichnet, daß man eine Nukleinsäure mit der Sequenz gemäß Tabelle 1 oder einer aufgrund der Degeneration des genetischen Codes davon abgeleiteten Sequenz, welche für PP9 kodiert, in ein Expressionssystem einbringt und dort zur Expression bringt.

2. Verfahren nach Anspruch 1, dadurch gekennzeichnet, daß als Expressionssystem prokaryotische Zellen eingesetzt werden.

3. Verfahren nach Anspruch 1, dadurch gekennzeichnet, daß als Expressionssystem eukaryotische Zellen eingesetzt werden.

4. Verfahren nach Anspruch 3, dadurch gekennzeichnet, daß als Expressionssystem Hefen eingesetzt werden.

5. Verfahren zur Herstellung eines Diagnostikums, dadurch gekennzeichnet, daß man eine Nukleinsäure mit der Sequenz gemäß Tabelle 1 oder einer aufgrund der Degeneration des genetischen Codes davon abgeleitete Sequenz, welche für PP9 kodiert, einsetzt.

**Claims**
**Claims for the following Contracting States : AT, BE, CH, DE, DK, FR, GB, IT, LI, LU, NL, SE**

1. A nucleotide sequence shown in Table 1, or a sequence derived therefrom on the basis of the degeneracy of the genetic code, coding for placenta-specific protein PP9.

2. A gene structure containing a nucleic acid as claimed in claim 1.

3. A vector containing a nucleic acid as claimed in claim 1.

4. Transformed cells containing a nucleic acid as claimed in claim 1.

5. A process for the preparation of PP9, which comprises a nucleic acid as claimed in claim 1 being introduced into an expression system and expressed therein.

6. A diagnostic aid which contains a nucleic acid as claimed in claim 1.

**Claims for the following Contracting State : ES**

1. A process for the preparation of PP9, which comprises a nucleic acid with the sequence shown in Table 1, or a sequence derived therefrom on the basis of the degeneracy of the genetic code, which codes for PP9 being introduced into an expression system and expressed therein.

2. The process as claimed in claim 1, wherein prokaryotic cells are used as expression system.

3. The process as claimed in claim 1, wherein eukaryotic cells are used as expression system.

4. The process as claimed in claim 3, wherein yeasts are used as expression system.

5. A process for producing a diagnostic aid, which comprises using a nucleic acid with the sequence shown in Table 1, or a sequence derived therefrom on the basis of the degeneracy of the genetic code, which codes for PP9.

**Revendications**
**Revendications pour les Etats contractants suivants : AT, BE, CH, DE, DK, FR, GB, IT, LI, LU, NL, SE**

1. Séquence nucléotidique selon le tableau 1 ou séquence dérivée de celle-ci en raison de la dégénérescence du code génétique, codant pour la protéine PP9 spécifique du placenta.

**2.** Structure génique contenant un acide nucléique selon la revendication 1.

**3.** Vecteur contenant un acide nucléique selon la revendication 1.

**4.** Cellules transformées, contenant un acide nucléique selon la revendication 1.

**5.** Procédé pour la production de PP9, caractérisé en ce que l'on introduit dans un système d'expression un acide nucléique selon la revendication 1 et on le conduit à l'expression dans celui-ci.

**6.** Agent de diagnostic, contenant un acide nucléique selon la revendication 1.

**Revendications pour l'Etat contractant suivant : ES**

**1.** Procédé pour la production de PP9, caractérisé en ce que l'on introduit dans un système d'expression un acide nucléique ayant la séquence selon le tableau 1 ou une séquence dérivée de celle-ci en raison de la dégénérescence du code génétique, qui code pour PP9, et on le conduit à l'expression dans ce système.

**2.** Procédé selon la revendication 1, caractérisé en ce que l'on utilise comme système d'expression des cellules procaryotes.

**3.** Procédé selon la revendication 1, caractérisé en ce que l'on utilise comme système d'expression des cellules eucaryotes.

**4.** Procédé selon la revendication 3, caractérisé en ce que, comme système d'expression, on utilise des levures.

**5.** Procédé pour la préparation d'un agent de diagnostic, caractérisé en ce que l'on utilise un acide nucléique ayant la séquence selon le tableau 1 ou une séquence dérivée de celle-ci en raison de la dégénérescence du code génétique, qui code pour PP9.

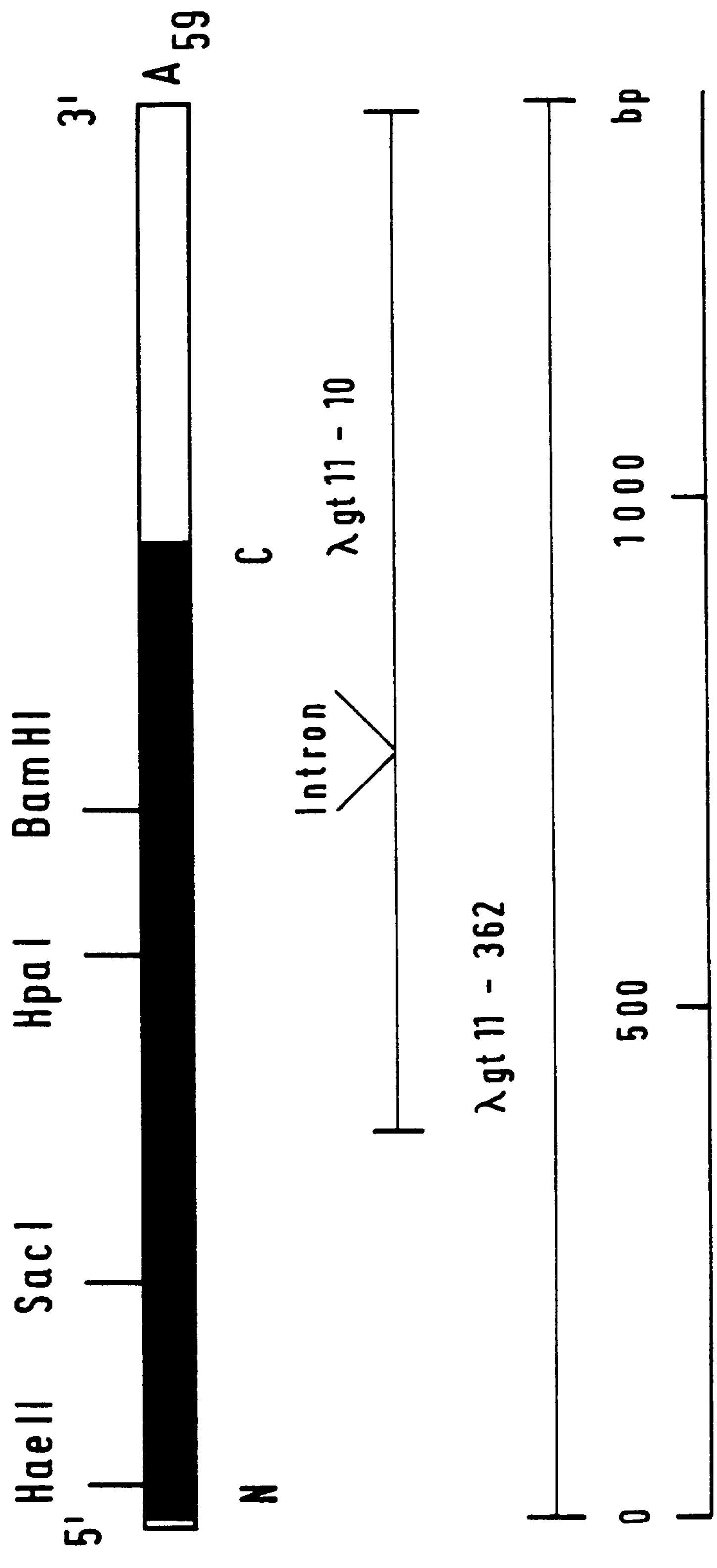
5'
HaeII
SacI
HpaI
BamHI
3'
A59
N
C
Intron
λgt 11 - 10
λgt 11 - 362
0
500
1000
bp